Europäisches Patentamt

European Patent Office

Office européen des brevets

(11) Publication number: **0 252 459**
**A1**

# EUROPEAN PATENT APPLICATION

(21) Application number: **87109648.3**

(51) Int. Cl.4: **A61K 9/70**

(22) Date of filing: **04.07.87**

(30) Priority: **07.07.86 US 882867**

(43) Date of publication of application:
**13.01.88 Bulletin 88/02**

(84) Designated Contracting States:
**AT BE CH DE ES FR GB GR IT LI LU NL SE**

(71) Applicant: **SCHERING CORPORATION**
**2000 Galloping Hill Road**
**Kenilworth, New Jersey 07033(US)**

(72) Inventor: **Zupon, Michael Anton**
**15 Hillside Avenue**
**Madison New Jersey 07940(US)**
Inventor: **Ambrosio, Thomas James**
**31 Eastern States Parkway**
**Somerville New Jersey 08876(US)**

(74) Representative: **von Kreisler, Alek,**
**Dipl.-Chem. et al**
**Deichmannhaus am Hauptbahnhof**
**D-5000 Köln 1(DE)**

(54) **Compartmentalized transdermal delivery system.**

(57) Compartmentalized transdermal delivery devices are disclosed.

FIG. 1

## COMPARTMENTALIZED TRANSDERMAL DELIVERY SYSTEM

The present invention relates to a pharmaceutical delivery system useful for the administration of formulations which are unstable on storage. In particular, it relates to a compartmentalized transdermal delivery device wherein two or more components of the transdermal formulation are provided separately in the device and mixed just prior to use.

Transdermal delivery devices are well known in the art as convenient and efficient means for controlled administration of drugs. A typical transdermal device comprises a backing layer defining one face surface, an adhesive layer defining the other face surface, which adhesive layer is protected until use by a release liner, and between the face surfaces a drug reservoir layer confined within wall members. The drug reservoir usually has an upper wall member (i.e., the upper wall member contiguous with the backing layer) and usually a lower wall member between the adhesive and the reservoir which may be a rate-controlling membrane, a non-rate controlling membrane or an impervious layer in which one or more wicks run between the reservoir and the adhesive. A reservoir may comprise a hollow container for holding liquid, or may comprise microcapsules or solid matrix material. The active drug may be present only in the reservoir, or a "priming dose" for immediate release may also be incorporated into the adhesive layer.

U.S. Patent 3,731,686 discloses transdermal devices comprising "at least one reservoir", but does not suggest that the contents of the reservoirs are to be combined prior to administration.

U.S. Patent 4,379,454 discloses devices with more than one reservoir wherein a percutaneous enhancer reservoir layer is separated from a drug reservoir-contact adhesive layer by a diffusion membrane.

European Patent Application 151,953 discloses mixing two phases, at least one of which contains the desired drug, to produce a supersaturated solution which may be administered in a transdermal device.

U.S. Patent 3,608,709 discloses multiple-compartment packages wherein the compartments are separated by breakable barriers.

Figure I is a longitudinal cross-sectional view of a transdermal device wherein two reservoirs (3, 3a) are defined by a wall member (I) and a membrane (4), which reservoirs are separated from each other by an impermeable but breakable membrane (2), wherein said wall member (I) is contiguous to a backing member (7) and said membrane (4) is contiguous to an adhesive layer (5), and wherein the adhesive layer (5) is protected by a readily removable release liner (6).

The present invention relates to a transdermal delivery device useful for transdermal applications wherein a drug-vehicle combination which exhibits the desired transdermal flux rates is physically or chemically unstable on storage. As illustrated in Figure I, an example of a transdermal device useful for the present invention comprises a backing member (7) defining one face surface, an adhesive layer (5) defining the other face surface, a release liner (6) covering the adhesive layer (5), an upper wall member (I) contiguous to the backing member and a rate-controlling or non-rate controlling membrane (4) contiguous to the adhesive layer. The upper wall member (I) and the membrane (4) define two or more reservoirs (3, 3a) separated from each other by impermeable but breakable membranes (2). The physically or chemically incompatible components of the formulation are provided in the separate reservoirs and just prior to patient use, the membrane separating the reservoirs is broken, e.g., by twisting or applying pressure to the device, and the contents of the reservoirs mixed e.g., by gently kneading the device between the fingers. The release liner (6) is removed, the transdermal device is applied to the skin of the patient and retained there by the adhesive (5), allowing the drug-vehicle combination to move from the reservoir through such means as a rate-controlling membrane or a non-rate controlling membrane (4), then through the adhesive and into the patient's skin. The device is worn for a specific period of time to permit the penetration of a desired amount of drug through the skin.

The device of the present invention may be used to separate a powder from a liquid (e.g., where the drug is unstable in the transdermal vehicle or carrier) or a liquid from another liquid (e.g., where the drug is stable in solution but not at the pH necessary for transdermal flux, or where the drug is unstable in the presence of a component of the formulation necessary for transdermal flux) during storage of the device. In the event that more than two components of the formulation are incompatible, multiple reservoirs may be used, e.g., powder/liquid/liquid, or powder/powder/liquid.

The use of particular components of the device, e.g., wall members, impermeable membranes and adhesives, is not critical to the practice of the invention. Thus, the invention contemplates the use of any components, including those not yet discovered or fully characterized, so long as said components can be used to form a transdermal device comprising two or more reservoirs. Examples of components useful in preparing devices of the present invention are as follows:

backing member: cellophane, polyester (e.g., polyethylene terephthalate), polyvinylidene chloride (e.g.,

2

Saran) and aluminum foil, coated or combined as a laminate;

upper wall member: polyethylene and copolymers of polyethylene, e.g., ethylene-vinyl-acetate or ethyl acrylic acid, or a polymeric based coating applied to the backing member.

rate or non-rate controlling wall membrane: polyolefins or copolymers thereof, e.g., polypropylene, polyethylene, polyethylene vinyl acetate, polyethyl acrylic acid, and polypropylene-polyethylene copolymer, homogeneous continuous films, or microporous or perforated films;

adhesive layer: a dermatologically acceptable permeable pressure-sensitive adhesive, e.g., an acrylic, silicone or rubber based adhesive;

release liner: silicone-treated polyester, silicone-treated paper, silicone treated foil, fluoro-carbon films, e.g., Teflon, Kel-F and aclar.

The backing member and the upper wall member are preferably laminated together, or, where the upper wall member or backing member is chosen appropriately to provide an impenetrable barrier, the other member, i.e., the backing member or the upper wall member, respectively, may be eliminated.

An impermeable breakable membrane dividing the device into two or more reservoirs may comprise a separate breaker strip, but preferably is formed by bonding, e.g., by heat-sealing, the impermeable wall member to the rate or non-rate controlling wall membrane along a line or lines extending transversely across the device. Techniques for preparing such bonds and for preparing transdermal devices in general are well known to those skilled in the art.

A preferred device of the present invention comprises a polyester backing member laminated to an upper wall member of polyethylene film and a rate or non-rate controlling wall membrane (i.e., a lower wall member) comprised of a copolymer of polyethylene (e.g., ethylene vinyl acetate-polyethylene copolymer) said lower wall member sealed (e.g., by application of heat and pressure) to the polyethylene film of said upper wall member to form two or more reservoirs. A preferred adhesive for coating the external surface of the lower wall member is an acrylic adhesive and a preferred release liner for protecting the adhesive layer until use comprises silicone treated polyester.

The characteristics or components of the drug-vehicle combination to be administered through the instant transdermal device are not critical to the invention. Such combinations may be liquids, lotions, creams or ointments, though for ease of mixing, less viscous formulations are preferred. Any compatible diluents, stabilizers, vehicles, gelling agents, preservatives, penetration enhancers and the like may be combined with the contents of any appropriate reservoir.

The length of time for which a device of the present invention is applied to the skin of the patient, the dosage size and the size of the device are similarly not critical to the practice of the invention, but are dependent on the particular drug and/or formulation being administered. The first device applied may be replaced if necessary with a new device, thereby providing a constant blood level of drug to the patient in need thereof.

The invention also contemplates a package which contains a specific number of transdermal devices that may be utilized to complete a specified course of treatment. For example, a package containing seven 24-hour transdermal devices would be utilized to complete a one week course of therapy.

A specific embodiment of the present invention relates to a transdermal delivery device for the administration of albuterol or a pharmaceutically acceptable salt thereof, a bronchodilator disclosed in U.S. Patent 3,644,353.

Aqueous mixtures of albuterol at pH 9 to 10 exhibit albuterol flux rates through human cadaver skin equivalent to that of albuterol solutions in dimethylsulfoxide (DMSO), a known skin permeation enhancer. See Table I:

## Table 1
### Albuterol Transdermal Flux

| Formulation | $n^a$ | Cumulative Albuterol Flux $(mg/cm^2)^b$ | | | |
|---|---|---|---|---|---|
| | | 6 hr | 12 hr | 24 hr | 48 hr |
| albuterol:water:Methocel® (20:79.5:0.5) (pH 9) | 3 | 0.07 | 0.22 | 0.57 | 1.4 |
| albuterol:DMSO (20:80) | 4 | 0.04 | 0.21 | 0.66 | 1.8 |

a – Number of Skin Penetration Cells

b – Mean of n Skin Penetration Cells

Skin Donor: F, W, 33

(Test conditions were similar to that described by Franz in _J. Invest. Derm._, _64_, 190 (1975)).

The above results indicate that the transdermal flux of albuterol from aqueous solution or neat DMSO is sufficient to achieve therapeutic albuterol plasma concentrations. However, DMSO is not an acceptable pharmaceutical vehicle and while aqueous albuterol formulations at pH 9 to l0 are pharmaceutically acceptable, they are unstable: a 20% aqueous suspension of albuterol at pH 9 showed a 20% loss of albuterol following storage for 30 days at 45°C. The compartmentalized transdermal device of the present invention therefore represents a solution to this instability problem: aqueous albuterol formulations may be administered via a two-reservoir transdermal device wherein the contents of one reservoir comprises albuterol (preferably undissolved or in solution at a pH wherein albuterol is stable) and the contents of the other reservoir comprises aqueous buffer at pH 9 to l0, and wherein upon mixing the contents of the two reservoirs, the pH of the albuterol solution is greater than pH 9. Typical buffer systems include glycerine/ sodium hydroxide and sodium borate/sodium hydroxide. Either or both reservoirs may contain additional components, e.g. viscosity imparting agents, anti-oxidants, coloring agents, preservatives and bulking agents.

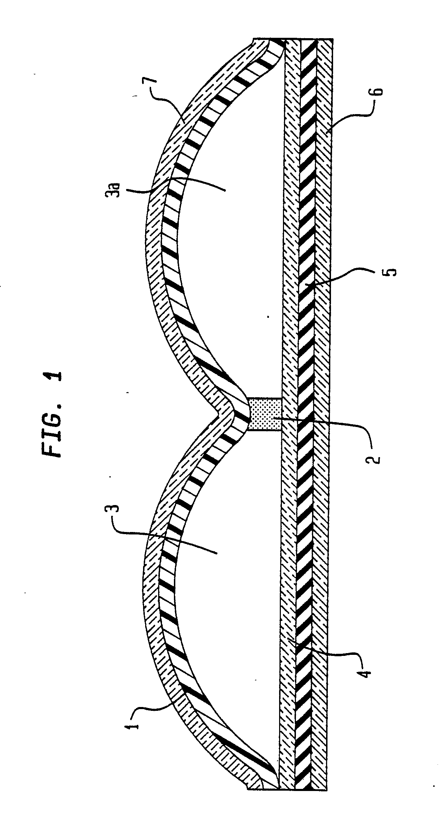

**Claims**

l. A transdermal delivery device comprising two or more reservoirs separated from each other by an impermeable but breakable membrane.

2. A transdermal delivery device of claim l comprising a backing member defining one face surface, an adhesive layer defining the other face surface, a release liner covering the adhesive layer, an upper wall member contiguous to the backing member and a rate-controlling or non-rate controlling membrane contiguous to the adhesive layer, wherein said wall member and said rate-controlling or non-rate controlling membrane define two or more reservoirs separated from each other by impermeable but breakable membranes.

3. A transdermal delivery device of claim 2 wherein the backing member is selected from cellophane, polyester, saran or aluminum foil.

4. A transdermal delivery device of claim 2 wherein the adhesive is a permeable, pressure sensitive, dermatologically acceptable adhesive selected from acrylic, silicone and rubber-based adhesives.

5. A transdermal delivery device of claim 2 wherein the upper wall member is a polyethylene or a copolymer of polyethylene film.

6. A transdermal delivery device of claim 2 wherein the rate or non-rate controlling membrane is a polyolefin or copolymer of polyolefin film.

7. A transdermal delivery device of claim 2 wherein the impermeable but breakable membrane is selected from polyolefins and copolymers of polyolefins.

8. A transdermal delivery device of claim 7 wherein the impermeable but breakable membrane is a copolymer of ethylene vinyl acetate.

# FIG. 1

| | DOCUMENTS CONSIDERED TO BE RELEVANT | | EP 87109648.3 | |
|---|---|---|---|---|
| Category | Citation of document with indication, where appropriate, of relevant passages | Relevant to claim | CLASSIFICATION OF THE APPLICATION (Int. Cl.4) | |
| D,X | EP - A2/A3 - 0 151 953 (BEECHAM GROUP PLC)<br><br>* Abstract; claims 1,9; pages 4,5 *<br><br>-- | 1-8 | A 61 K 9/70 | |
| D,A | US - A - 3 608 709 (B.R.PIKE)<br><br>* Abstract; column 3, lines 37-60; fig. 2,4a,4b *<br><br>-- | 1,3-8 | | |
| A | WO - A1 - 82/03 174 (MERCK PATENT GESELLSCHAFT MIT BESCHRÄNKTER HAFTUNG)<br><br>* Abstract; fig. 10 *<br><br>---- | 1 | | |
| | | | TECHNICAL FIELDS SEARCHED (Int. Cl.4) | |
| | | | A 61 K 9/00<br>B 65 D | |

The present search report has been drawn up for all claims

| Place of search | Date of completion of the search | Examiner |
|---|---|---|
| VIENNA | 02-10-1987 | HERING |